# FASCICULE DE BREVET EUROPEEN

(11) **EP 0 775 492 B1**
(45) Date de publication et mention de la délivrance du brevet: **27.12.2000**
(21) Numéro de dépôt: 96402180.2
(22) Date de dépôt: 14.10.1996
(51) Int. Cl.: A61K 33/30, A61K 33/32, A61K 33/24, A61K 33/00

(54) **Utilisation de sel de lanthanide, d'étain, de zinc, de manganèse, d'yttrium, de cobalt, de baryum, de strontium dans une composition pour la peau**
Verwendung von Lanthanide-, Zinn-, Mangan-, Yttrium-, Kobalt-, Barium-, Strontiumsalzen in einem Hautpflegemittel
Use of salts of lanthanide, tin, zinc, manganese, yttrium, cobalt, strontium in skin composition

(30) Priorité: 26.10.1995 FR 9512656
(43) Date de publication de la demande: 28.05.1997
(73) Titulaire: L'OREAL, 75008 Paris (FR)
(72) Inventeur: de Lacharriere, Olivier, 75015 Paris (FR); Breton, Lionel, 78000 Versailles (FR)
(74) Mandataire: Lhoste, Catherine

(56) Documents cités:
- EP-A- 0 085 579
- EP-A- 0 135 312
- EP-A- 0 217 975
- EP-A- 0 250 300
- EP-A- 0 388 275
- WO-A-91/01624
- WO-A-91/02538
- WO-A-96/19182
- WO-A-96/19183
- WO-A-96/19184
- FR-M- 5 394
- US-A- 4 711 780
- US-A- 5 202 130
- DATABASE WPI Section Ch, Week 9612 Derwent Publications Ltd., London, GB; Class A96, AN 96-114522 XP002009056 & RU 2 038 072 C (ORG CHEM TECHN RES INST) , 27 Juin 1995

## Description

La présente invention se rapporte à l'utilisation d'au moins un sel de lanthanides, de manganèse, d'étain, de zinc ou d'yttrium, de cobalt, de baryum, de strontium dans une composition dermatologique ou pharmaceutique comme antagoniste de substance P, pour la préparation d'une composition pharmaceutique ou dermatologique, pour traiter les douleurs associées à l'un au moins des désordres cutanés, y compris le cuir chevelu et les muqueuses, suivants : zona, post-zostérienne, brûlure, démodécide, ulcère cutané, fibrose, et/ou contrôler la cicatrisation hypertrophique et/ou traiter la couperose.

Il existe chez les mammifères des polypeptides appartenant à la famille des tachykinines qui induisent sur les fibres musculaires lisses des contractions rapides. Parmi les composés de cette famille on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de très nombreux désordres sont associés à un excès de synthèse et / ou de libération de substance P.

La substance P intervient notamment dans la transmission de la douleur (dentaire, cutané, tympanique) et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie, maladies psychotiques), dans des maladies respiratoires (telles que par exemple les broncho-pneumonie, toux, emphysème, bronchiolite) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn, gastrites, gastro-entérites, spasticites intestinales), dans des désordres cutanés (tels que par exemple le psoriasis, les maladies prurigineuses, l'herpès, les photodermatoses, les dermatites atopiques, les dermatites de contact, les lichens, les prurigos, les prurits, les rosacées, les peaux sensibles, les dartres, les érythèmes solaires et émotionnels, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Raynaud), dans des désordres immunologiques, dans des troubles du tractus urinaire et ou génital (tels que par exemple l'incontinence, la cystite), dans des maladies rhumatismales dans certaines maladies dermatologiques (telles que l'eczéma) et dans les affections ophtalmologiques (telles que par exemple les conjonctivites, les uvéites, les prurits oculaires, les blépharites, les douleurs oculaires et les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P. Particulièrement, pour qu'une substance soit reconnue comme un antagoniste de substance P, elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus et sont cités dans de nombreux documents.

Cependant aucun de ces documents n'envisage, ni ne suggère qu'un sel de lanthanide, de zinc, d'yttrium, d'étain, de cobalt, de baryum, de strontium puisse avoir une activité antagoniste de substance P telle que définie ci-dessus et donc puisse être notamment utilisé pour traiter les douleurs associées au désordres cutanés indiqués ci-dessus.

En outre, il est connu du document FR-M-5394 un médicament contenant des crésols et du chlorure de strontium pour augmenter les propriétés antibactériennes et antimicrobiennes de ces crésols. Toutefois, ce document ne décrit ni ne suggère que le chlorure de strontium puisse diminuer voire éliminer, à lui seul, les symptômes tels que les irritations, douleurs, démangeaisons, picotements, causés par une quelconque maladie, ni diminuer voire supprimer ces mêmes symptômes causés par un produit irritant.

La demanderesse a découvert qu'un sel choisi parmi les sels de lanthanide, de manganèse, d'étain, de zinc, d'yttrium, de cobalt, de baryum, de strontium répond aux caractéristiques définies comme caractérisant un antagoniste de substance P.

L'invention concerne l'utilisation d'au moins un sel choisi parmi les sels de lanthanide, de manganèse, d'étain, d'yttrium, de baryum, de strontium et leurs associations pour la préparation d'une composition dermatologique ou pharmaceutique destinée à traiter les douleurs associées à l'un des désordres cutanés y compris le cuir chevelu et les muqueuses suivants : zona, douleurs post-zostériennes, brûlure, démodécie, ulcère cutané, fibrose, et/ou contrôler la cicatrisation hypertrophique et/ou traiter la couperose.

L'invention concerne aussi l'utilisation d'au moins un sel choisi parmi les sels de cobalt, de zinc ou leur association pour la préparation d'une composition dermatologique ou pharmaceutique destinée à traiter les douleurs associées à l'un des désordres cutanés y compris le cuir chevelu et les muqueuses, suivants : zona, douleurs post-zostériennes, brûlure, démodécie, ulcère cutané, fibrose, et/ou traiter la couperose.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium .

L'invention s'applique à tous les désordres cutanés ci-dessus, liés à un excès de synthèse et/ou de libération de substance P.

Les sels utilisés peuvent être par exemple des carbonates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate).

Les sels peuvent être choisis avantageusement parmi les sels de zinc, de baryum, de manganèse, d'yttrium, de strontium et leurs mélanges.

De préférence, on utilise des chlorures et des nitrates avantageusement de zinc, de gadolinium, d'yttrium et de strontium.

De façon préférée, le sel est un sel de strontium.

Selon l'invention, le sel peut être utilisé en une quantité allant de 10⁻⁵ % à 20 % du poids total de la composition et préférentiellement en une quantité allant de 10⁻² % à 15 % du poids total de la composition et mieux de 0,5 à 8% du poids total de la composition.

Le sel peut être utilisé dans une composition qui doit être ingérée, injectée localement ou appliquée sur la peau (sur toute zone cutanée du corps), les cheveux, les ongles. Selon le mode d'administration, cette composition peut se présenter sous toutes les formes galéniques normalement utilisées.

Pour une application topique sur la peau, la composition peut avoir la forme notamment de solution aqueuse, hydroalcoolique ou huileuse ou de suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, de microémulsions ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, ou de mousses.

Pour l'injection locale, la composition peut se présenter sous forme de lotion aqueuse, de suspension huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes de fond de teint, crèmes anti-solaires), des fonds de teint fluides, des laits corporels de protection ou de soin, des laits après-solaires, des lotions, gels ou mousses pour le soin de la peau, comme des lotions de nettoyage, des lotions après-solaires, des compositions pour le bain, des compositions désodorisantes comprenant un agent bactéricide, des gels ou lotions après-rasage, des compositions contre les piqûres d'insectes, des compositions anti-douleur, des compositions pour traiter certaines maladies de la peau comme l'eczéma, la rosasée, le psoriasis, les lichens, les prurits sévères et celles énoncées précédemment.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

Le sel utilisé selon l'invention peut aussi être incorporé dans diverses compositions pour soins capillaires, et notamment des shampooings, des lotions traitantes, des lotions restructurantes pour les cheveux, des lotions ou des gels antichute, des shampooings antiparasitaires, etc.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans les domaines considérés. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition peut contenir également des adjuvants habituels dans les domaines considérés, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras, des acides gras (acide stéarique), des cires d'abeilles, de carnauba, et de la paraffine.

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux ou bactériens et l'amidon.

Comme actifs lipophiles, on peut utiliser le rétinol (vitamine A) et ses dérivés, le tocophérol (vitamine E) et ses dérivés, les acides gras essentiels, les céramides, les huiles essentielles.

Selon l'invention on peut, entre autres, associer aux sels de lanthanide, de manganèse, d'étain, de zinc, d'yttrium, de cobalt, de baryum, de strontium d'autres agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les antiseptiques ;
- les antimétabolites.

Ainsi, selon un mode particulier, l'invention se rapporte à l'utilisation d'au moins un sel choisi parmi les sels de lanthanide, de manganèse, d'étain, de zinc, d'yttrium, de cobalt, de baryum, de strontium et leur mélanges dans une composition comprenant au moins un agent choisi parmi les agents antibactériens, antiparasitaires, antifongiques, antiviraux anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

De façon avantageuse, selon l'invention au moins un sel de lanthanide, de manganèse, d'étain, de zinc, d'yttrium, de cobalt, de baryum, de strontium peut être associé à des produits à effet irritant utilisés couramment dans le domaine dermatologique ou pharmaceutique, produits qui sont parfois des actifs dermatologiques ou pharmaceutiques. La présence d'un antagoniste de substance P sous la forme d'au moins un sel de lanthanide, de manganèse, d'étain, de zinc, d'yttrium, de cobalt, de baryum, de strontium pour la fabrication d'une composition dermatologique ou pharmaceutique comprenant un produit ayant un effet irritant permet d'atténuer fortement, voire de supprimer cet effet irritant. Cela permet en outre d'augmenter la quantité d'actif à effet irritant par rapport à la quantité d'actif normalement utilisée, en vue d'une efficacité améliorée.

L'invention peut être mise en oeuvre notamment en appliquant les compositions hygiéniques, dermatologiques ou pharmaceutiques telles que définies ci-dessus, selon la technique d'utilisation habituelle de ces compositions. Par exemple : application de crèmes, de gels, de sérums, de lotions, de compositions après-solaires sur la peau ou sur les cheveux secs, application d'une lotion pour cheveux sur cheveux mouillés, de shampooings.

Les exemples et compositions suivants illustrent l'invention sans la limiter aucunement. Dans les compositions les proportions indiquées sont des pourcentages en poids.

| Crème de nettoyage (Formule A) | |
|---|---|
| Carbonate d'yttrium | 5,00 |
| Alcool Cétylique | 2,00 |
| Stéarate de Glycérol | 2,00 |
| Acide Stéarique | 2,00 |
| Polyglyceryl-3 Hydroxylauryl Ether | 5,00 |
| Huile Minérale Codex | 12,00 |
| Carbomer | 0,35 |
| Hydroxyde de Sodium | 0,15 |
| Parfum qsp | |
| Méthyl Paraben | 0,20 |
| Eau déminéralisée Stérile qsp | 100,00 |

| Lait de nettoyage (Formule B) | |
|---|---|
| Nitrate de lanthane | 0,5 |
| Carbomer | 0,40 |
| Hydroxyde de Sodium | 0,10 |
| Huile Minérale Codex | 5,00 |
| Stéarate de Glycérol | 1,00 |
| Alcool Cétylique | 0,50 |
| PEG 100 Stéarate | 0,80 |
| Méthyl Paraben | 0,20 |
| Parfum qsp | |
| Eau déminéralisée Stérile qsp | 100,00 |

| Lotion de soin (Formule C) | |
|---|---|
| Chlorure de cobalt | 0,5 |
| Glycérol | 2,00 |
| Méthyl Paraben | 0,15 |
| Parfum qsp | |
| Eau déminéralisée Stérile qsp | 100,00 |

| Crème de soin (Formule D) | |
|---|---|
| Chlorure de baryum | 1,00 |
| Stéarate de Glycérol | 1,00 |
| PEG 100 Stéarate | 1,00 |
| Acide Stéarique | 1,00 |
| Alcool Cétylique | 2,00 |
| Huile de Soja | 3,00 |
| huile de Palme | 2,00 |
| Cyclométhicone | 2,00 |
| Diméthicone | 1,00 |
| Polyacrylamide | 0,20 |
| Glycérol | 3,00 |
| Méthyl Paraben | 0,20 |
| Parfum qsp | |
| Eau Stérile Déminéralisée qsp | 100,00 |

| Gel pour le soin du visage (Formule E) | |
|---|---|
| Chlorure de strontium | 5,00 |
| Hydroxypropylcellulose (Klucel H vendu par la société) Hercules) | 1,00 |
| Antioxydant | 0,05 |
| Isopropanol | 40,00 |
| Conservateur | 0,30 |
| Eau qsp | 100 % |

| Crème de soin de l'érythème solaire (émulsion huile-dans-eau) (Formule F) | |
|---|---|
| Aspartate de zinc | 0,75 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau qsp | 100 % |

| Lotion pour éliminer les cicatrices (Formule G) | |
|---|---|
| Glycérophosphate de strontium | 1,5 |
| Acide glycolique | 50,00 |
| Hydroxypropylcellulose (Klucel H) | 0,05 |
| NaOH qsp | pH=2,80 |
| Ethanol qsp | 100 % |
| Conservateur | 0,30 |

## Revendications

1. Utilisation d'au moins un sel choisi parmi les sels d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, d'étain, de manganèse, de baryum ou de strontium et leur association pour la fabrication d'une composition pharmaceutique ou dermatologique pour traiter les douleurs associées à l'un au moins des désordres cutanés suivants : zona, douleurs post-zostériennes, brûlure, démodécie, ulcère cutané, fibrose, et/ou contrôler la cicatrisation hypertrophique et/ou traiter la couperose.

2. Utilisation selon la revendication précédente, caractérisée en ce que le sel est choisi parmi les sels de baryum, de manganèse, d'yttrium, de strontium ou leur mélange.

3. Utilisation selon l'une quelconque des revendications précédentes, caractérisée en ce que le sel est un sel de strontium.

4. Utilisation d'au moins un sel choisi parmi les sels de cobalt, de zinc ou leur association pour la préparation d'une composition dermatologique ou pharmaceutique destinée à traiter les douleurs associées à l'un des désordres cutanés suivants : zona, douleurs post-zostériennes, brûlure, démodécie, ulcère cutané, fibrose, et/ou traiter la couperose.

5. Utilisation selon la revendication 4, caractérisée en ce que le sel est un sel de zinc.

6. Utilisation selon l'une quelconque des revendications 1 à 5, caractérisée en ce que les sels sont constitués d'ions choisis parmi les ions chlorure, borate, bicarbonate, carbonate, nitrate, hydroxyde, sulfate, glycérophosphate, les acides de fruit, les acides aminés.

7. Utilisation selon la revendication 6, caractérisée en ce que les ions sont les ions chlorures, nitrates.

8. Utilisation selon l'une quelconque des revendications précédentes, caractérisée en ce que le sel est utilisé en une quantité allant de 10⁻⁵ % à 20 % du poids total de la composition.

9. Utilisation selon l'une quelconque des revendications précédentes, caractérisée en ce que le sel est utilisé en une quantité représentant de 10⁻² % à 15 % du poids total de la composition.

10. Utilisation selon l'une quelconque des revendications précédentes, caractérisée en ce que la composition comprend de plus au moins un actif dermatologique ou pharmaceutique différent des sels.

11. Utilisation selon la revendication 10, caractérisée en ce que la composition contient, en outre, au moins un actif choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

12. Utilisation selon l'une quelconque des revendications précédentes, caractérisée en ce que la composition se présente sous forme d'une solution aqueuse, huileuse, hydroalcoolique, d'une émulsion huile-dans-eau ou eau-dans-huile, d'une microémulsion, d'un gel aqueux ou anhydre, d'un sérum, d'une dispersion de vésicules.

## Patentansprüche

1. Verwendung mindestens eines Salzes, das unter den Yttriumsalzen, Lanthansalzen, Cersalzen, Praseodymsalzen, Neodymsalzen, Promethiumsalzen, Samariumsalzen, Europiumsalzen, Gadoliniumsalzen, Terbiumsalzen, Dysprosiumsalzen, Holmiumsalzen, Erbiumsalzen, Thuliumsalzen, Ytterbiumsalzen, Lutetiumsalzen, Zinnsalzen, Mangansalzen, Bariumsalzen oder Strontiumsalzen oder deren Gemischen ausgewählt ist, zur Herstellung einer pharmazeutischen oder dermatologischen Zusammensetzung zur Behandlung von Schmerzen, die bei mindestens einer der folgenden Störungen der Haut beteiligt sind: Gürtelrose, Post-Zoster-Schmerzen, Verbrennungen, Haarbalgmilbenbefall, Hautgeschwüre und Fibrose, und/oder zur Steuerung der hypertrophen Narbenbildung und/oder zur Behandlung von Teleangiektasien.

2. Verwendung nach dem vorhergehenden Anspruch, dadurch gekennzeichnet, daß das Salz unter den Bariumsalzen, Mangansalzen, Yttriumsalzen, Strontiumsalzen oder deren Gemischen ausgewählt ist.

3. Verwendung nach einem der vorhergehenden Ansprüche, dadurch gekennzeichnet, daß das Salz ein Strontiumsalz ist.

4. Verwendung mindestens eines Salzes, das unter den Cobaltsalzen, Zinksalzen oder deren Gemischen ausgewählt ist, zur Herstellung einer dermatologischen oder pharmazeutischen Zusammensetzung zur Behandlung von Schmerzen, die bei mindestens einer der folgenden Störungen der Haut beteiligt sind: Gürtelrose, Post-Zoster-Schmerzen, Verbrennungen, Haarbalgmilbenbefall, Hautgeschwüre und Fibrose, und/oder zur Behandlung von Teleangiektasien.

5. Verwendung nach Anspruch 4, dadurch gekennzeichnet, daß das Salz ein Zinksalz ist.

6. Verwendung nach einem der Ansprüche 1 bis 5, dadurch gekennzeichnet, daß die Salze Ionen enthalten, die unter den Chloriden, Boraten, Bicarbonaten, Carbonaten, Nitraten, Hydroxiden, Sulfaten, Glycerophosphaten, Fruchtsäureionen und Aminosäureionen ausgewählt sind.

7. Verwendung nach Anspruch 6, dadurch gekennzeichnet, daß es sich bei den Ionen um Chloride und Nitrate handelt.

8. Verwendung nach einem der vorhergehenden Ansprüche, dadurch gekennzeichnet, daß das Salz in einem Anteil von 10⁻⁵ bis 20 % des Gesamtgewichts der Zusammensetzung verwendet wird.

9. Verwendung nach einem der vorhergehenden Ansprüche, dadurch gekennzeichnet, daß das Salz in einem Anteil von 10⁻² bis 15 % des Gesamtgewichts der Zusammensetzung verwendet wird.

10. Verwendung nach einem der vorhergehenden Ansprüche, dadurch gekennzeichnet, daß die Zusammensetzung ferner mindestens einen dermatologischen der pharmazeutischen Wirkstoff enthält, der von den Salzen verschieden ist.

11. Verwendung nach Anspruch 10, dadurch gekennzeichnet, daß die Zusammensetzung ferner mindestens einen Wirkstoff enthält, der unter den antibakteriellen Mitteln, antiparasitären Mitteln, Antimykotika, antiviralen Mitteln, entzündungshemmenden Mitteln, antipruritischen Mitteln, Anästhetika, Keratinolytika, Mitteln gegen freie Radikale, Antiseborrhöika, Antischuppenmitteln, Mitteln gegen Akne und/oder Mitteln, die die Differenzierung und/oder Proliferation und/oder Pigmentierung der Haut modulieren, ausgewählt ist.

12. Verwendung nach einem der vorhergehenden Ansprüche, dadurch gekennzeichnet, daß die Zusammensetzung als wäßrige, ölige oder wäßrig-alkoholische Lösung, Öl-in-Wasser- oder Wasser-in-Öl-Emulsion, Mikroemulsion, wasserfreies oder wäßriges Gel, Serum oder Vesikeldispersion vorliegt.

## Claims

1. Use of at least one salt chosen from yttrium, lanthanum, cerium, praseodymium, neodymium, promethium, samarium, europium, gadolinium, terbium, dysprosium, holmium, erbium, thulium, ytterbium, lutetium, tin, manganese, barium or strontium salts and a combination thereof for the manufacture of a pharmaceutical or dermatological composition for treating pain associated with at least one of the following skin disorders: zona, poszoster pain, scald, demodicidosis, skin ulcer, fibrosis and/or controlling hypertrophic cicatrisation and/or treating acne rosacea.

2. Use according to the preceding claim, characterized in that the salt is chosen from barium, manganese, yttrium or strontium salts or a mixture thereof.

3. Use according to any one of the preceding claims, characterized in that the salt is a strontium salt.

4. Use of at least one salt chosen from cobalt and zinc salts or a combination thereof for the preparation of a dermatological or pharmaceutical composition intended for treating pain associated with one of the following skin disorders: zona, poszoster pain, scald, demodicidosis, skin ulcer, fibrosis and/or controlling hypertrophic cicatrisation and/or treating acne rosacea.

5. Use according to Claim 4, characterized in that the salt is a zinc salt.

6. Use according to any one of the Claims 1 to 5, characterized in that the salts consist of ions chosen from chloride, borate, bicarbonate, carbonate, nitrate, hydroxide, sulphate, and glycerophosphate ions, fruit acids, amino acids.

7. Use according to Claim 6, characterized in that the ions are chloride or nitrate ions.

8. Use according to any one of the preceding claims, characterized in that the salt is used in a quantity ranging from 10⁻⁵ % to 20 % of the total weight of the composition.

9. Use according to one of the preceding claims, characterized in that the salt is used in a quantity representing from 10⁻² % to 15 % of the total weight of the composition.

10. Use according to one of the preceding claims, characterized in that the composition comprises, in addition, at least one dermatological or pharmaceutical active agent different from the salts.

11. Use according to Claim 10, characterized in that the composition contains, in addition, at least one active agent chosen from antibacterial, antiparasitic, antifungal, antiviral, anti-inflammatory, antipruritic, anaesthetic, keratolytic, anti-free radical, antiseborrhoeic, antidandruff, or anti-acne agents and/or skin pigmentation and/or proliferation and/or differentiation modulating agents.

12. Use according to one of the preceding claims, characterized in that the composition is provided in the form of an aqueous, oily or aqueous-alcoholic solution, an oil-in-water or water-in-oil emulsion, a microemulsion, an aqueous or anhydrous gel, a serum, or a dispersion of vesicules.
